# EUROPEAN PATENT APPLICATION

(11) **EP 3 839 966 A1**
(43) Date of publication of application: **23.06.2021**
(21) Application number: 20150386.9
(22) Date of filing: 06.01.2020
(51) Int. Cl.: G16H 40/60, G16H 50/20, G16H 50/50

(54) **SYSTEM FOR CONFIGURING PATIENT MONITORING**

(30) Priority: 19.12.2019 US 201962950541 P
(71) Applicant: Koninklijke Philips N.V., 5656 AG Eindhoven (NL)
(72) Inventor: BULUT, Murtaza, 5656 AE Eindhoven (NL); LAVEZZO, Valentina, 5656 AE Eindhoven (NL); COX, Lieke Gertruda Elisabeth, 5656 AE Eindhoven (NL); HENDRIKS, Cornelis Petrus, 5656 AE Eindhoven (NL)
(74) Representative: Steenbeek, Leonardus Johannes

(57) **Abstract**

A system (10) is for configuring operating settings and parameters of a patient monitor (14), in particular a bedside patient monitor, based on use of a digital twin (32) of the at least one patient (13) being monitored. In some embodiments, the system (10) configures settings of a signal processing module (16) of the patient monitor, the signal processing module adapted to process incoming sensor data to extract or derive one or more clinical parameters relating to the patient. Outputs of the digital twin can relate to a current or predicted future state of the patient and thus can intelligently guide the signal processing module to extract and output clinical parameters which are most clinically relevant to the patient's condition at any given time. Thus the patient monitor (14) automatically displays the most clinically relevant parameters for the patient at each given moment, which allows a clinician to rapidly ascertain the clinical state of the patient, and to take any necessary action quickly to treat the patient. Medical outcome can be expected to be improved as a result.

## Description

### FIELD OF THE INVENTION

The present invention relates to a system for configuring patient monitoring with a patient monitor, in particular a bedside patient monitor.

### BACKGROUND OF THE INVENTION

Patient monitors, for example bedside patient monitors, are used to monitor key clinical parameters related to a patient, for example physiological or anatomical parameters, and to display trended measurements of the parameters on a display for a clinician to view. For example, vital signs can be measured and displayed in real time on the monitor. The measured clinical parameters are typically continuously analyzed by the patient monitor, and in the case that parameters fall outside of some pre-defined limits, an alarm may be triggered to alert clinical staff. Clinical intervention can then take place to remedy the situation and normalize the clinical parameters.

Bedside patient monitors are typically coupled to a set of medical sensors collecting physical data relating to the patient. These can include medical biosensors and mechanical sensors. This input sensor data is used by the patient monitor to determine or derive the clinical parameters for display on the display unit.

Patient monitors typically also comprise a signal processing component for processing raw sensor data and converting it into a format that can be shown on the display device or transferred to an external display or recording device. In some cases, it may process the sensor data to compute from it one or more parameters which are not directly observable.

Patient monitors typically also comprise a local display device. This is typically in the form of a digital display screen for displaying clinical parameter signals or data.

The data displayed on the patient monitor display can for example comprise both "original" or direct sensor signals, and computed signals. The original or direct signals are signals indicative of a parameter which is directly observed by one of the sensors, e.g. temperature or heart rate, e.g. an ECG signal indicative of heart rate. A computed signal or parameter is a parameter that is derived through processing or computation performed using sensor data received from one or more sensors. For example, it may include a maximum, minimum or average value of a measured (or derived) parameter or may reflect a physiological parameter which none of the sensors directly observes.

A limitation of state of the art patient monitors is that the clinical parameters which are extracted and displayed on the monitor screen are typically static, or must be manually selected for display by a user in accordance with preference. This can lead to important information being missed if a particularly clinically relevant parameter is not part of the selected parameters for extraction or for display. It may also lead to time being wasted by clinicians in adjusting the signal extraction and display settings in order to view the clinical parameter that is needed. This time delay can have significant consequence for the patient in terms of medical outcome, especially if the delay occurs during a medical emergency.

It is possible to consider a more intelligent interface, wherein the parameters which are displayed are automatically adjusted based on analysis of values of the parameters, for example if they exceed one or more thresholds.

However, a bedside patient monitor is naturally limited in the sophistication of analysis it can perform of incoming signals. This means that the analysis may typically be erroneous or misleading, leading to displayed parameters not accurately reflecting the most clinically relevant for the patient. In particular, it is difficult to provide such functionality which is able to accurately take account of the specific medical context of a given patient. This may well lead to further delay or inconvenience for clinicians if erroneous parameters are displayed. This could have a negative impact on medical outcome. In many cases, the automated functionality may simply be switched off by a clinician to avoid inconvenience.

### SUMMARY OF THE INVENTION

Improvements to bedside patient monitors for remedying one or more of the above described problems would therefore generally be of advantage. The invention is defined by the independent claims. The dependent claims define advantageous embodiments.

As will be outlined below, embodiments of the present invention are based on application of a digital twin of the monitored patient to configure settings of the patient monitor. Outputs of the digital twin may be used to configure which clinical parameters are extracted from incoming sensor data and/or to configure which parameters are displayed at any given time on the monitor display, and/or which are given most prominence on the display.

For example, according to examples in accordance with one aspect of the invention, there is provided a system for configuring patient monitoring by a patient monitor unit, the patient monitor unit arranged in use to receive input sensor data from one or more patient sensors, and operable to display clinical information related to a monitored patient based on the sensor data; the patient monitor unit comprising a signal processing module configured to apply signal processing to the input sensor data to generate one or more information outputs related to the monitored patient; the system comprising a processor arrangement communicatively coupled to a data storage arrangement storing a digital model of at least part of an anatomy of the monitored patient, and further coupleable in use with one or more patient sensors to receive patient sensor data, and the digital model configured for simulating an actual physical state of said at least part of the anatomy based on development of the model using the sensor data.

Outputs of the digital model are used to configure settings or operation parameters of the patient monitor unit.

In accordance with one set of advantageous embodiments, the processor arrangement is adapted to configure the signal processing performed by the signal processing module based at least in part on outputs from the digital model. For example, it configures settings or parameters of the signal processing. Different examples and options for configuring the signal processing will be described in more detail below.

Embodiments of the invention are based on use of a personal digital model of at least a portion of the anatomy of the patient, and in preferred embodiments are based on use of such a digital model for guiding signal processing performed by a signal processing component of the patient monitor. For example, outputs of the digital model may guide which set of patient parameters are extracted from the data and displayed, or may guide the methods used to extract the parameters.

The patient digital model referred to above provides a digital twin of the patient. It may provide a model of the overall physical or clinical state of the patient, encompassing multiple anatomical regions. The personal digital model is configured to provide a real-time replica of the state of the patient, based on real-time sensor data. Such a digital model is known in the art as a digital twin since it simulates an actual physical state of the patient in real time or approximately real time. The digital model may therefore be otherwise referred to as a digital twin in this disclosure.

A digital twin is operable to simulate an actual real time state of a patient, and is operable through biophysical modelling embodied in the digital twin to determine parameters relating to the patient which may not be directly observable. It can comprise both bio-physical and data driven models. It is also able to generate predictions regarding a future state of the patient, including predicted future pathologies or diseases. By utilizing such outputs of a digital twin, this enables a patient monitor to intelligently extract from incoming sensor data the patient parameters which are most clinically important, both for the present condition of the patient, and the future condition of the patient. Accordingly, signal processing and extraction by the patient monitor can be more intelligently tailored to the medical need of the patient, providing a user of the patient monitor with the parameters most relevant to the current or predicted future state of the patient.

The signal processing may be configured for example based on simulations of the digital twin and/or predictions of the digital twin.

The system may include the patient monitor or the system may connect to a patient monitor which is not part of the system and be operable to control parameters of the patient monitor, e.g. parameters of the signal processing.

The information outputs generated by the patient monitor preferably relate to a current clinical state of the patient. They may include clinical parameters related to a current state of the patient. For example, they may include one or more physiological or anatomical parameters.

In one or more embodiments, the selection of information outputs to be derived by the signal processing module may be determined based on outputs from the digital twin.

For example, the selection of clinical parameters which are derived or computed by the signal processing module may be configured based on outputs from the digital model.

The digital twin itself may be configured for generating proposals for information outputs to derive based on information derived from its simulations, for example based on information related to a current or future clinical state of the patient.

Alternatively, the system (e.g. the processor arrangement) may be configured to determine which information outputs to derive, based on information from the digital twin relating to a current or future clinical state of the patient.

Additionally or alternatively, in one or more embodiments, a selection of one or more signal processing methods that are applied for deriving a given one or more information outputs may be configured based in part on outputs from the digital twin. Here, the methods by which the signal processing module derives information outputs, e.g. the algorithm or computation method applied to derive the outputs, may be configured or selected or controlled based on output from the digital twin.

Additionally or alternatively, parameters or settings of one or more signal processing methods applied by the signal processing module may be configured based on outputs of the digital twin. For example, a precision or accuracy level of one or more signal processing methods might be adjusted, or a required sampling frequency of input data for the method might be adjusted. These represent examples only.

In accordance with one or more embodiments, the digital model may be operable to generate one or more outputs related to a current or predicted future clinical state of the at least part of the anatomy of the patient.

The digital model may be operable to generate one or more outputs related to a current or predicted future pathology of the at least part of the anatomy of the patient.

In accordance with one or more embodiments, the digital model may be operable to generate one or more prediction outputs pertaining to a predicted future state of the at least part of the anatomy.

The signal processing may be configured based at least in part on any of said outputs or prediction outputs.

The prediction outputs may relate to any predicted future pathology of said at least part of the anatomy.

The signal processing may be configured such that the information outputs are relevant to said predicted future pathology.

The digital model may additionally or alternatively be configured to generate one or more outputs indicative of current pathologies of the patient. The signal processing module may be controlled to derive clinical parameters from the input sensor data relevant to those pathologies.

In accordance with one or more embodiments, information outputs generated by the signal processing module may include one or more clinical parameters of the patient, for example one or more physiological or anatomical parameters of the patient.

The information outputs preferably include signal outputs, for example representative of a clinical (e.g. physiological or anatomical) parameter as a function of time.

In some examples, the information outputs may additionally or alternatively include one or more discrete values, for example a calculated value for a particular physiological parameter or an estimated measurement of a particular anatomical feature or entity or body, or for instance an average, maximum or minimum value of a directly measured parameter.

In accordance with one or more embodiments, the digital model may be operable to generate one or more outputs indicative of physiological or anatomical parameters of the patient. These parameter outputs may be computed by the digital model based on one or more inputs, e.g. sensor inputs. They may relate to parameters which are not directly observable, but which can be derived or calculated using the digital model.

In some embodiments, the clinical information displayed by the patient monitor is based in part on said outputs of the digital model.

The signal processing of the signal processing module may be applied to these outputs of the digital model in combination with the sensor data. Hence, outputs of the digital model may in some cases be provided as inputs to the signal processing of the signal processing module for generating the one or more information outputs. Thus the one or more information outputs of the signal processing module may be based in part on outputs of the digital model in some cases.

These outputs of the digital twin provided to the patient monitor unit may include signal outputs, for example representative of a clinical (e.g. physiological or anatomical) parameter as a function of time.

The processor arrangement referred to above may be coupled to the same set of patient sensors as the patient monitor, or may be coupled to a different set of patient sensors. The processor arrangement may be arranged to receive sensor data from only a subset of the patient sensors feeding the patient monitor, and/or may receive input from extra sensors to the patient monitor, and vice versa.

The information outputs may be directly or indirectly based on the input sensor data (and/or input parameter data generated by the digital twin, as mentioned above). For example, the information output may be directly indicative of the physical measurement that the sensor detects or that the digital twin output represents, e.g. temperature or pulse rate. It may instead be indicative of a different parameter, e.g. one not directly observable and which is computed or calculated through further processing of the input, or by combining multiple inputs. For example, certain hemodynamic parameters can be derived through application of a computation algorithm using for example blood pressure and ECG data.

In accordance with one or more embodiments, one or more of the information outputs generated by the signal processing module may be supplied as respective information inputs to the digital twin. This may enhance the simulations performed by the digital twin.

In accordance with one or more embodiments, the selection of clinical information displayed on the patient monitor display may be configured based at least in part on outputs from the digital model. In preferred examples, it may be configured based on one or more outputs of the digital twin indicative of a current or future clinical state of the patient.

For example, based on a derived current or predicted future clinical state of the patient, it may be determined that one or more parameters are of more immediate significance than others. The clinical parameters displayed on the screen may be selected to be those determined as the most important. The selection of parameters displayed on the screen may be dynamically updated at regular intervals based on change in patient clinical state as determined by the digital twin.

This has clinical benefits for the patient, since the clinician using the patient monitor is shown the most clinically relevant parameters automatically when attending to the patient. This potentially saves time wasted by the clinician in searching for the parameter they need to medically attend to the patient, and also ensures that the clinician does not miss inadvertently any parameter which may be important to the patient's current or near future status. Medical outcome for the patient can be expected to be improved as a result.

In accordance with one or more embodiments, the patient monitor unit may comprise more than one display unit, and optionally wherein a different selection of information outputs is displayed on each of the display units. For example, the display units may be at different locations relative to the patient, e.g. one display for viewing by the patient, one at the foot of the bed for viewing by a passing clinician, and/or one at the end of the row of beds for viewing by a supervising clinician. The selection of information outputs displayed on each display, and/or the relative sizing and/or position of the different items of clinical information may vary between different of the display units.

In accordance with one or more embodiments, the display size at which each element of clinical information is displayed on the patient monitor display may be determined at least in part on outputs from the digital model. Preferably it is determined based on one or more outputs of the digital model indicative of a current or future clinical state of the patient.

For example, based on a derived current or future clinical state of the patient, it may be determined that one or more parameters are of more immediate significance than others. The more important parameters may be rendered at a larger size on the screen, or otherwise with greater visibility or prominence.

In preferred examples, the display size is proportional to the determined clinical importance of the parameter to the digital twin-determined patient current or future clinical state.

In this way, the observer of the display, e.g. clinician, is effectively provided additional information in the form of an importance ranking of each of a set of clinical parameters. This information is conveyed in a natural and intuitive manner, in the form of different sizing of different clinical parameters. This additional information has clinical benefits for the patient, since it means that the clinician can recognize quickly which parameters are the most important to keep watch of, and also ensures that the most clinically important parameters for the patient's current or future state are most easily observable or accessible to the clinician which may improve response time to any urgent clinical event such as a deterioration in condition. Medical outcome for the patient can be expected to be improved as a result.

Additionally or alternatively, an order in which clinical information is displayed on the screen may be configured at least in part on outputs from the digital model, for example outputs related to a current or future clinical state of the patient. The order may be based on a determined ranking of clinical importance or relevance of the parameters, based on information from the digital twin. For example, the order of display may follow the determined order of importance, with the most important at the highest position on the display and declining in position with determined importance ranking.

In accordance with one or more embodiments, the selection of clinical information displayed on the display of the patient monitor unit and/or the display size at which each element of clinical information is displayed on the patient monitor may be updated recurrently based on changes in said outputs of the digital model.

In accordance with one or more embodiments, a frequency at which simulations are run on the digital twin may be adjusted recurrently based in part on outputs of the digital twin. For example, the frequency may be adjusted based on a current or predicted future clinical state of the patient determined by the digital model.

In accordance with one or more embodiments, the display settings of the patient monitor display (e.g. which information outputs are displayed, or the size or position of display (as discussed above)) and/or the signal processing of the signal processing module may be configured based at least in part on the frequency of simulation runs. For example, results from more frequently run simulations may in some example be emphasized less on the display, and results from less frequent simulations may be emphasized more, or vice versa depending on the current or predicted patient condition.

For example, for a stable patient less frequent simulations might carry more clinical weight, while for an unstable patient, more frequent simulation results might be considered of greater clinical relevance.

In accordance with one or more embodiments, the system may further include a communication interface for communicating with one or more remote systems or devices, for instance for communicating the derived information outputs to a central monitoring unit arranged for displaying patient data for a plurality of patients, or for communicating with a central server or system for outputting derived information outputs and/or for receiving stored patient medical data, e.g. medical history data about the monitored patient.

In advantageous embodiments, the patient monitor unit may include an alarm means for generating a sensory alarm signal to alert a user, e.g. clinician, based on the derived information outputs meeting one or more alarm criteria, such as exceeding a defined alert threshold. In advantageous examples, the alarm criteria are configured based in part on outputs from the digital twin. For example, one or more thresholds for the alarm trigger may be set based on information from the digital twin.

In accordance with one set of embodiments, the system may comprise the patient monitor unit. In some examples, the processor arrangement and data storage arrangement may be comprised by the patient monitor unit, for example incorporated as components within the patient monitor unit. Alternatively, the processor arrangement and data storage arrangement may be separate from the patient monitor unit.

Examples in accordance with a further aspect of the invention provide a method of configuring patient monitoring by a patient monitor unit, the patient monitor unit arranged in use to receive input sensor data from one or more patient sensors, and operable to display clinical information related to a monitored patient based on the sensor data, the patient monitor unit comprising a signal processing module configured to apply signal processing to the input sensor data to generate one or more information outputs related to the monitored patient.

The method comprises retrieving a digital model of at least part of an anatomy of the monitored patient, and developing the model based on input sensor data from one or more patient sensors so as to simulate within the model an actual physical state of said at least part of the anatomy of the patient. The method further comprises configuring parameters or operation settings of the patient monitor unit based on outputs of the digital model.

In an advantageous set of embodiments, the method comprises in particular configuring the signal processing performed by the signal processing module based at least in part on outputs from the digital model.

Implementation options and details for each of the above steps may be understood and interpreted in accordance with the explanations and descriptions provided above for the apparatus aspect of the present invention (i.e. the system aspect).

Any of the examples, options or embodiment features or details described above in respect of the apparatus aspect of this invention (in respect of the system) may be applied or combined or incorporated into the present method aspect of the invention.

Examples in accordance with a further aspect of the invention provide a computer program product comprising code means configured when run on a processor, to cause the processor to perform the method in accordance with any embodiment set out above or described below, or in accordance with any claim of this application.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows in block diagram form an example system in accordance with one or more embodiments;
Fig. 2 schematically illustrates example inputs and outputs of an example digital model (digital twin) suitable for use in accordance with one or more embodiments; and
Fig. 3 schematically illustrates example information flows between components of an example system in accordance with one or more embodiments.

### DETAILED DESCRIPTION OF THE EMBODIMENTS

The invention will be described with reference to the Figures. It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for configuring operating settings and parameters of a patient monitor, in particular a bedside patient monitor, based on use of a digital twin of the at least one patient being monitored. In some embodiments, the system configures settings or parameters of a signal processing module of the patient monitor, the signal processing module adapted to process incoming sensor data to extract or derive one or more clinical parameters relating to the patient. Outputs of the digital twin can relate to a current or predicted future state of the patient and thus can intelligently guide the signal processing module to extract and output clinical parameters which are most clinically relevant to the patient's condition at any given time. Thus the patient monitor automatically displays the most clinically relevant parameters for the patient at each given moment, which allows a clinician to rapidly ascertain the clinical state of the patient, and to take any necessary action quickly to treat the patient. Medical outcome can be expected to be improved as a result.

In order to enable faster and potentially more informative assessment and treatment of the patient condition, embodiments propose adapting one or both of the signal processing of incoming sensor data and/or display of patient monitor data, based on information provided by a digital twin concerning current and/or predicted future patient condition. Adaptation of the patient monitor functionality may include calculation of new clinical parameters, and/or adjusting the ranking and emphasis of the clinical signals on the display screen, and/or predicting future patient conditions and adapting the patient monitor behavior accordingly. In some embodiments, the qualifications and expertise of a staff member observing the patient monitor may be taken into account.

A limitation of the state of the art patient monitors and how they are used is that the same clinical parameters are always displayed and always in the same order and format, which may result in clinically important information being missed (e.g. due to staff fatigue and/or alert fatigue). It can also slow down ascertaining of relevant clinical information as a clinician may need to manually change the clinical parameters that are being displayed in order to observe the parameter that is needed to assess the patient condition. This can impact medical outcome for the patient.

In order to enable faster and potentially more informative assessment and treatment of the patient condition, it is proposed in embodiments to adapt the patient monitor operating settings based on information from a digital twin of the patient and potentially also to include clinical information about the patient derived by the digital twin on the patient monitor display. Thus the digital twin can augment the clinical information derived directly by the patient monitor itself.

As discussed, embodiments of the invention are based on application of digital twin technology to enhance the functionality of a patient monitor, e.g. a bedside patient monitor. In particular, the data about a patient which is extracted, derived and/or displayed on the patient monitor can be dynamically adjusted and configured based on insights generated by the digital twin. A digital twin is operable to simulate an actual real time state of a patient, and is operable through biophysical modelling embodied in the digital twin to determine parameters relating to the patient which may not be directly observable. It is also able to generate predictions regarding a future state of the patient, including predicted future pathologies or diseases. This enables a patient monitor to intelligently derive and display the patient parameters which are most clinically important, both for the present condition of the patient, and the future condition of the patient.

To better understand the invention, a brief background introduction to the concept of digital twins will now be outlined.

The digital twin concept is a fairly recent development in technology. In this concept, a digital representation (the digital twin) of a physical system is provided and connected to its physical counterpart, for example through the Internet of things as explained in US 2017/286572 A1. Through this connection, the digital twin typically receives data pertaining to the state of the physical system, such as sensor readings or the like, based on which the digital twin can predict the actual or future status of the physical system, e.g. through simulation, as well as analyze or interpret a status history of the physical twin. It essentially provides a digital replica of the physical object, which permits for example monitoring and testing of the physical object without needing to be in close proximity to it. In case of electromechanical systems, this for example may be used to predict the end-of-life of components of the system, thereby reducing the risk of component failure as timely replacement of the component may be arranged based on its end-of-life as estimated by the digital twin.

Digital twins are most typically used to represent mechanical or electrical devices such as manufacturing machines or even aircraft. Such digital twins are useful to monitor functioning of a device and schedule maintenance for example.

Such digital twin technology is also becoming of interest in the medical field, as it provides an approach to more efficient medical care provision. For example, the digital twin may be built using imaging data of the patient, e.g. a patient suffering from a diagnosed medical condition as captured in the imaging data, as for instance is explained by Dr Vanessa Diaz in https://www.wareable.com/health-and-wellbeing/doctor-virtual-twin-digital-patient-ucl-887 as retrieved from the Internet on 29 October 2019. Such a digital twin may serve a number of purposes. Firstly, the digital twin rather than the patient may be subjected to a number of virtual tests, e.g. treatment plans, to determine which treatment plan is most likely to be successful to the patient. This therefore reduces the number of tests that physically need to be performed on the actual patient.

The digital twin of the patient for instance further may be used to predict the onset, treatment or development of such medical conditions of the patient using a patient-derived digital model, e.g. a digital model that has been derived from medical image data of the patient. In this manner, the medical status of a patient may be monitored without the routine involvement of a medical practitioner, e.g. thus avoiding periodic routine physical checks of the patient. Instead, only when the digital twin predicts a medical status of the patient indicative of the patient requiring medical attention based on the received sensor readings may the digital twin arrange for an appointment to see a medical practitioner to be made for the patient. This typically leads to an improvement in the medical care of the patient, as the onset of certain diseases or medical conditions may be predicted with the digital twin, such that the patient can be treated accordingly at an early stage, which not only is beneficial to the patient but can also reduce (treatment) costs. Moreover, major medical incidents that the patient may be about to suffer may be predicted by the digital twin based on the monitoring of the patient's sensor readings, thereby reducing the risk of such incidents actually occurring. Such prevention avoids the need for the provision of substantial aftercare following such a major medical incident, which also alleviates the pressure on a healthcare system otherwise providing such aftercare.

In addition to representing patients, digital twins may also in some applications be used to represent medical devices and equipment.

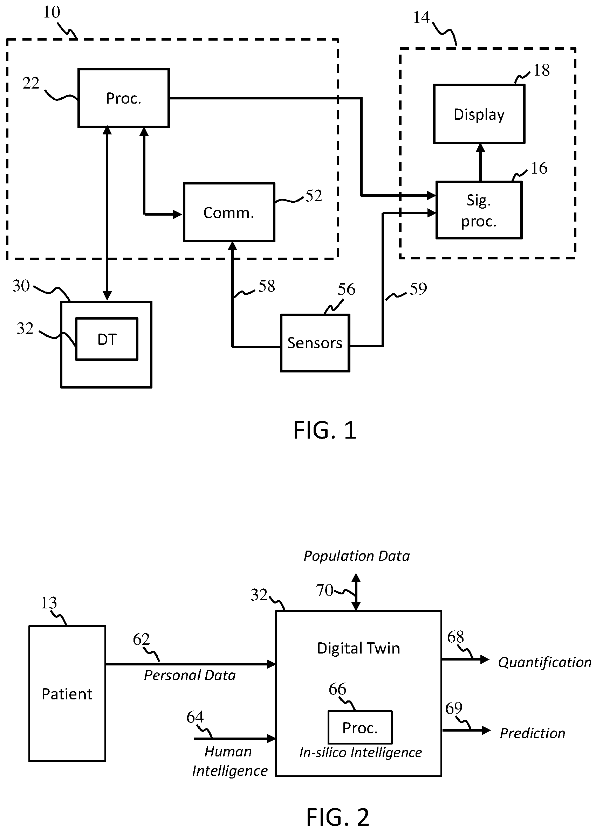

Fig. 1 schematically outlines in block diagram form an example system 10 in accordance with one or more embodiments of the present invention.

The system 10 comprises a processor arrangement 22 communicatively coupled to a data storage arrangement 30 storing a digital model 32 ("digital twin 32") of at least part of an anatomy of the monitored patient.

The system is arranged to be coupleable in use with one or more patient sensors 56 to receive patient sensor data 58.

The digital model 32 is configured for simulating an actual physical state of said at least part of the anatomy of the patient based on development of the digital model using the sensor data 58. The patient sensors may monitor one or more clinical parameters of the patient, for example one or more physiological or anatomical parameters, for example one or more vital signs. By way of non-limiting example, the parameters monitored by the sensors 56 may include one or more of: heart rate, SpO₂, breathing rate, cardiac output, temperature. The sensors may also include imaging sensors, for example ultrasound or optical sensors for imaging one or more anatomical regions of the body.

In the example shown in Fig. 1, the digital data storage arrangement 30 is not part of the system 10 itself, but is external to it. The system is arranged to be communicable with the data storage arrangement in use. In other embodiments however, the data storage arrangement 30 may be included as part of the system 10.

In the example shown in Fig. 1, the patient sensors 56 are not part of the system 10 itself, but are external to it. The system is arranged to be communicable with the sensors in use. In other embodiments however, the sensors may be included as part of the system.

As illustrated in Fig. 1, the one or more patient sensors 56 may in some examples be communicatively coupled to the processor arrangement 22 via an intermediary communication module 52 which provides a communication interface. This may for instance comprise a wired or wireless communication link for communicatively linking the sensors and processor arrangement 22. It may comprise a local area network, an Internet connection, or any other form of connection or link. However, a communication interface is not essential and the sensors may be directly coupled to the processor arrangement. Also, in further examples, the one or more patient sensors 56 may additionally or alternatively be communicatively coupled directly with the digital twin itself.

The system 10 is arranged to be operatively coupleable in use with a patient monitor unit 14 the patient monitor unit arranged in use to receive input sensor data 59 from one or more patient sensors 56, and operable to display on a display unit or device 18 clinical information related to the monitored patient based on the sensor data 59.

The patient monitor unit 14 comprises a signal processing module 16 configured to apply signal processing to the input sensor data 59 to generate one or more information outputs related to the monitored patient. The clinical information displayed on the patient monitor display unit 18 may include at least a portion of the information outputs generated by the signal processing module, or all of the information outputs in some examples. The display unit 18 may additionally display further information from other sources, for example from a central patient database, e.g. relating to patient medical history, or previous recorded values for one or more clinical parameters, or from the digital twin 32 itself in some examples (e.g. communicated via the processing unit).

In the example of Fig. 1, the same set of sensors is coupled to, and provides sensor data to, both the system 10 (and the digital model 32) and to the patient monitor unit 14. However, in further embodiments, each of the patient monitor 14 and the system (and digital twin) may be arranged coupled to a different (dedicated) set of patient sensors. In some embodiments, the patient monitor 14 and processor arrangement (and digital model 32) may share at least a subset of the same physical sensors but wherein there are additional sensors feeding additional information to the processor arrangement 22 (and digital model 32) compared to the patient monitor, or vice versa.

The processor arrangement 22 is adapted to configure the signal processing performed by the signal processing module 16 of the patient monitor unit 14 based at least in part on outputs from the digital model 32.

In the example shown in Fig. 1, outputs from the digital model 32 are communicated to the signal processing module 16 via the processor arrangement 22. However, in other examples, outputs of the digital model may be directly coupled to the signal processing module, or may be coupled to the optional communication interface 52, and communicated to the signal processing module 16 via the communication interface.

The signal processing module may be implemented in any number of ways. For example, it may comprise one or more processors or microcontrollers for performing the signal processing. In some examples, its functionality may be integrated within a broader central processing module of the patient monitor unit, so that the signal processing module is merely a functional module rather than a separate physical module.

In the example of Fig. 1, the patient monitor unit 14 is not part of the system 10, but is external to it, and wherein the system 10 is arranged to be operatively coupled with the patient monitor unit 14 in use. For example, the processor arrangement of the system may be incorporated in a controller unit which connects to the patient monitor, and which is able to communicate information to the patient monitor and preferably to control aspects of functionality of the patient monitor. For example, the connection between the two may permit the transmission of control commands or signals from the processor arrangement 22 to the patient monitor unit, for example for configuring the signal processing performed by the signal processing module 16, or in other embodiments, for configuring information display settings for the display unit 18.

In further embodiments however, the system 10 may include the patient monitor unit 14. For example, the system 10 may be a patient monitoring system which includes the components of the patient monitor unit 14 in addition to at least the processor arrangement 22 of the system 10 shown in Fig. 1. In other words, the processor arrangement 22 and the patient monitor unit 14 form a single integrated system or unit. In some examples, the processor arrangement 22 may be integrated in the patient monitor unit.

In accordance with advantageous embodiments, the system 10 (for example the processor arrangement 22 of the system) is arranged to recurrently or continuously update or develop the patient digital model 32 based on the sensor data 58 received from the patient sensors 56 in order to keep the model as an up-to-date live replica or representation of the real-time physical state of the patient (anatomy).

The processor arrangement 22 of the system 10 may take any suitable shape. The processor arrangement may for example comprise one or more processors, processor cores or the like that cooperate to form such a processor arrangement. It may consist of a single component, or its functions may be distributed among a plurality of processing components.

Similarly, the communication module 52 (where one is included) may take any suitable shape, such as a wireless or wired data communication module, as is well known in the art and will therefore not be further explained for the sake of brevity only. Furthermore, although in Fig. 1, the communication module is shown as a separate component, this is merely schematic, and the communication module may be merely a functional module. Its function may be performed by a separate component, or its function may be performed by the processor arrangement itself or by another component of the system.

The digital model 32 in the remainder of this application may also be referred to as a digital twin of the patient 10. Such a digital twin typically provides a model of both the elements and the dynamics of the at least portion of the anatomy of the patient (i.e. the physical twin). The digital twin may by way of example integrate artificial intelligence, machine learning and/or software analytics with spatial network graphs to create a 'living' digital simulation model of the at least portion of the patient's anatomy. By way of non-limiting example, the at least portion of the patient's anatomy may be a part of a lumen system of the patient (e.g. vascular or digestive systems), such that the digital twin comprises a model of this part of a lumen system of the patient 10. Such a living digital simulation may for example involve the use of a fluid dynamics model, a systemic model, a tissue deformation model and/or a fluid-structure interaction model in order to develop or update the digital twin based on received sensor data 58 indicative of parameters of a physical state of the patient.

In other words, the sensor data 58 provided by the one or more sensors 56 may be used to update and change the digital twin dynamically, and in real time, such that any changes to the patient 10 as highlighted by the sensor data are reflected in the digital twin. As such, the digital twin forms a learning system that learns from itself using the sensor data provided by the one or more sensors 56. The digital twin is thus a dynamic model which dynamically develops or updates so as to provide an accurate representation of the patient's real anatomy.

The digital twin 32 typically encompasses one or more biophysical models which are used to model the anatomy represented by the model. The digital twin may also encompass one or more data driven models, meaning models which are able to estimate or model aspects of a state of the patient based on input data such as patient history data, or family medical history data, or data regarding comparative population samples for example. This may use modelling algorithms separate from the biophysical models. The two types of models together provide a powerful arrangement for modelling the patient.

The digital twin 32 of the patient may be initially developed from patient data, e.g. imaging data such as CT images, MRI images, ultrasound images, and so on. A typical workflow for creating and validating a 3D, subject-specific biophysical model is depicted in "Current progress in patient-specific modeling", by Neal and Kerckhoff, 1, 2009, Vol. 2, pp. 111-126. For example, in case of a digital twin representing part of the cardiovascular system of the patient, such a biophysical model may be derived from one or more angiograms of the patient. For example, the sensor data produced by the patient sensors 56 may be used to continuously or periodically update the boundary condition of a flow simulation through the digital lumen model (i.e. the digital twin) of the patient.

In operation, the processor arrangement 22 may develop or update the digital twin 32 using the received patient sensor 56 data in order to simulate the actual physical state of the at least portion of the anatomy of the patient.

Development and implementation of digital twin models for various example applications are described in the literature for this field. By way of example, implementation details for various example digital twin models are described in the following papers: González, D., Cueto, E. & Chinesta, F. Ann Biomed Eng (2016) 44: 35; Ritesh R. Rama & Sebastian Skatulla, Towards real-time cardiac mechanics modelling with patient-specific heart anatomies, Computer Methods in Applied Mechanics and Engineering (2018) 328; 47-74; Hoekstra, A, et al, Virtual physiological human 2016: translating the virtual physiological human to the clinic, interface Focus 8: 20170067; and "Current progress in patient-specific modeling", by Neal and Kerckhoff, 1, 2009, Vol. 2, pp. 111-126. Details are also outlined in "Computational Biomechanics for Medicine", Grand R. Joldes et al, Springer.

In general, the digital model, e.g. of an organ or tissue area of the patient, incorporates a number of different (e.g. heterogeneous) material properties as parameters of the model, which may include blood vessels, muscles, fat, lining tissue, bones, calcified areas, which each have specific (biomechanical) material properties. These material properties form parameters for the model to allow an evolution of a physical state of the patient to be accurately simulated.

The fundamentals of a patient-specific digital model for a given patient's anatomy may be developed in advance of operation of the system 10, such that before the digital twin is put into use as part of patient monitoring, the digital model is an accurate representation of the current physical state of the portion of the anatomy of the patient to be monitored, and incorporates sufficient information and knowledge about the material properties and physical response characteristics to allow the model to be dynamically evolved or developed based on the received sensor data 58.

The parameter values may be obtained for instance from literature and mapped onto the model, or obtained directly from measurements, e.g. elastography, performed on the patient.

As mentioned, the digital twin can comprise both bio-physical and data driven models. In general, a digital model 32 (digital twin) of a patient may enable advanced interactive visualization and physical insights of relevant health information of a patient. Combining visualization with predictive models that provide projections of future health status and outcome of medical interventions can be used to provide personalized clinical decision support (CDS) to improve diagnostics, treatment selection and interventions across the health continuum. For most accurate prediction, digital twins integrate biophysical models (0D - 4D), data analytics, image processing and clinical expertise.

Features and properties of the digital twin for use in accordance with embodiments of the present invention will now be described in more detail.

As discussed, a digital twin 32 can be considered as a "living" digital copy of a real object, such as a patient (or portion of a patient's anatomy, such as an organ) being monitored by the patient monitor.

One example implementation of a digital twin suitable for use in accordance with one or more embodiments is schematically outlined in Fig. 2.

The digital twin 32 is schematically shown as receiving a number of possible model inputs, and generating a number of outputs. The digital twin 32 may be provided information inputs 62 representative of personal data related to the patient 13, meaning data representative of clinically relevant parameters or properties pertaining to the specific patient being monitored. It is this information that permits the digital model to be kept a personalized digital model, with model parameters tuned to model the personal clinical characteristics of the patient being monitored.

The personal data inputs 62 may include, by way of non-limiting example, patient sensor data, medical imaging data, and/or patient medical record data (e.g. EMR data).

The digital twin 32 may further be provided user-provided or defined information inputs 64. These may be referred to as "human intelligence" inputs. These may include by way of non-limiting example: simulation parameter settings, input data requirements for the simulation (e.g. a minimum level of data or a minimum set of data sources or data points), simulation settings (e.g. resolution or precision level vs speed of simulation), and usage planning (e.g. planned frequency of simulation runs, and clinical purpose for the simulation, e.g. which clinical pathologies are of most interest).

The digital twin 32 may be further provided with information inputs 70 representative of medical data or information from comparative populations of patients, e.g. past medical history data or treatment information related to other patients belonging to one or more populations which are clinically relevant to the current patient. For example, a particular population or cohort might be selected who share a minimum subset of clinical details with the patient being monitoring, e.g. same key medical pathologies, similar medical progression, similar past treatment records. The population data may include information indicating clinical parameter data for the patients over time, treatment courses for patients in the selected populations or cohorts, and corresponding medical progression. This may inform modelling by the digital twin of expected evolution of the physical state of the patient in the event that certain clinical parameter values or trends are measured for the patient or certain treatments administered for example.

In the example of Fig. 2, the digital model 32 comprises its own processor component 66 for running simulations for simulating the actual physical state of the at least portion of the patient. This can be referred to as "in-silico intelligence".

This processing component incorporates one or more biophysical and/or data driven model algorithms which are used to run simulations. The processor component 66 of the digital twin 32 is arranged to receive the various model inputs 62, 64, 70 and to run the modelling to simulate a state of the patient. From these simulations, one or more model outputs can be derived or extracted. For example, outputs can be derived relating to one or more physiological or anatomical parameters of the anatomy being modelled. These can be current values for certain parameters or they can be predicted future values for one or more parameters. They can be in the form of signal outputs in some examples, e.g. signal outputs indicative of parameter values as a function of time.

Although the digital twin is shown as comprising its own processing component 66, in further examples, the functionality of this processing component may be performed by the processor arrangement 22 of the system 10 shown in Fig. 1 for example. In all cases, a processing means is needed to execute the functionality of the digital twin, but these can be configured in different ways. In Fig. 1, the processor arrangement 22 may perform the processing of the digital twin models, and the data storage arrangement stores the model algorithms and associated data for example. In the example of Fig. 2, the digital twin is shown schematically as a self-contained component or module, encompassing the processor component (and also a memory) itself. Both arrangements are possible. In some examples, the processor components 62 may be understood as being provided by the processor arrangement 22, i.e. they may be understood as referring to one and the same component.

The processor component 22 may encompass, by way of non-limiting example, machine learning and artificial intelligence algorithms, deep learning algorithms, biophysical modelling and data driven modelling.

As mentioned, one or more model outputs can be derived or extracted from the digital twin 32. These may include quantification outputs 68, related to current values for one or more clinical parameters, and preferably also prediction outputs 69 related to predicted future values of one or more clinical parameters. Qualitative outputs may also be generated, such as for instance a qualitative indication of a stability of patient condition (e.g. stable / unstable).

By way of example, the quantification outputs 68 can include past and current status of the portion of the anatomy of the patient, or of the patient as a whole, and/or can include information indicative of a longitudinal risk assessment of the patient. These are examples only.

The prediction outputs 69 can include predictions regarding a future status of the at least portion of the anatomy or of the patient as a whole, predictions as to medical outcome of the patient, and may also include one or more recommendations as to medical actions to take to improve medical outcome or future progression of the patient status.

It is emphasized that the above example represents just one example arrangement for a digital twin and one example set of possible model inputs and outputs. Additional or different inputs may be provided and/or outputs derived from the digital twin in other examples.

As discussed above, in accordance with a preferred set of embodiments, the processor arrangement is adapted to configure parameters of the signal processing performed by the signal processing module 16 of the patient monitor unit 14 based at least in part on outputs from the digital model 32 (or digital twin). Thus the digital twin can be used to guide the signal processing of the patient monitor unit.

As mentioned above, the signal processing module 16 is configured to apply signal processing to the input sensor data 59 to generate one or more information outputs related to the monitored patient 13. These information outputs relate to clinical information for the patient, for example signals or values representative of one or more clinical parameters for the patient, e.g. physiological or anatomical parameters. These may be in some examples statistical values computed from input sensor measurements, e.g. an average or maximum or minimum value of certain sensor measurements, or may be parameters which are not directly observable, but can be computed (estimated) by processing one or more inputs sensor measurements according to a particular processing method.

Both the particular selection of information outputs which are generated by the signal processing module 16 and the methods by which they are derived can be configured. This configuration may be advantageously informed by outputs from the digital twin relating to a current or future state of the patient for example.

Thus, in at least one set of embodiments, the selection of information outputs to be derived by the signal processing module 16 may be determined based on outputs from the digital model 32.

Additionally or alternatively, the selection of one or more signal processing methods to be applied for deriving a given one or more information outputs may be determined based in part on outputs from the digital model. Thus here, the digital twin is used to guide the selection of the signal processing method.

Additionally or alternatively, parameters or settings of one or more signal processing methods applied for deriving a given one or more information outputs may be determined based in part on outputs from the digital model.

For example, if the digital twin 32 predicts a particular patient condition or pathology (e.g. internal bleeding) will develop in the near future, then a certain group of patient specific parameters clinically relevant to treating and avoiding this condition developing can be chosen to be extracted. For example, a baseline of past sensor data over a previous period of time can be computed for a set of parameters including e.g. mean arterial pressure, heart rate, heart-rate variability, respiration rate, SpO₂. A cross-correlation between all these may be calculated in addition. These parameters may be the most clinically relevant for normalizing the current measurements (e.g. to abate the internal bleeding, or to avoid the internal bleeding developing if detected early enough), and performing differential analyses.

Preferably also, the processing methods used to compute each of these information outputs (and/or parameters or settings of the methods) can be determined based on the digital twin output.

For example, the digital twin prediction may be used to determine which computation method to apply (e.g. whether to compute HRV as the standard deviation of the inter-beat intervals or as the ratio of low frequency regions to high frequency regions) as well as for instance parameters or thresholds of these methods (e.g. cutoff points for the mentioned frequency regions). As another example, the digital twin 32 may run simulations to determine which frequency bands are expected to be the most clinically significant or relevant to focus on and visualize (e.g. for processing of an ECG or EEG signal).

By way of example, in some cases, a new method of signal processing might become available or possible due to additional information provided in outputs of the digital twin. The system may be configured to adapt the signal processing methods that are applied at least in part dependent upon which outputs from the digital twin are available.

For example, there might be stored locally or remotely a database of possible signal processing methods, and the information inputs required in order to use each. Depending upon the digital twin outputs generated, application of these methods becomes feasible or not feasible.

By way of a more specific example, a case might be considered in which deriving an information output by the signal processing module requires a raw input sensor signal to be decomposed into decomposition signals (signals of the same length as the original, but e.g. filtered to different component frequencies, or component signal characteristics). When there is relatively little information available from the digital twin, decomposition techniques may be applied which are purely driven by the raw signal characteristics such as empirical mode decomposition (EMD). When more information from the digital twin is available, more complex methods may be applied, e.g. the signal may be first filtered (based on the digital twin output), and then EMD applied. With the additional digital twin information, a wavelet function might be constructed (e.g. fitted to the output of digital twin), and the wavelet utilized for decomposition. Use of a specific wavelet decomposition instead of EMD is one example of selection of different signal processing techniques or methods dependent upon the outputs of the digital twin.

The above represents just one illustrative example, and various other examples will also be apparent to the skilled person.

Which information outputs are extracted and /or the processing methods used may be determined by the digital twin 32 itself and provided as one or more outputs. Alternatively, the outputs of the digital twin may simply relate to parameters pertaining to current or predicted future status or pathologies of the patient, and wherein the processor arrangement 22 (or another component of the system 10) utilizes these outputs to inform determination of which information outputs are derived and/or the methods for deriving them.

Thus in accordance with some embodiments, the digital model is operable to generate one or more outputs related to a current or predicted future clinical state of the at least part of the anatomy of the patient. The model may be operable to generate one or more outputs related to a current or predicted future pathology of the at least part of the anatomy of the patient.

The digital model may be operable to generate one or more prediction outputs pertaining to a predicted future state of the at least part of the anatomy, and wherein said parameters of the signal processing are configured based at least in part on said prediction outputs.

The mentioned prediction outputs may indicate any predicted future pathology that the digital twin has identified of said at least part of the anatomy. The parameters of the signal processing of the signal processing unit 16 may then be configured such that the information outputs relate to said predicted future pathology.

As mentioned, the information outputs generated by the signal processing module 16 may typically include one or more clinical parameters of the patient, for example one or more physiological or anatomical parameters of the patient.

As discussed, embodiments of the invention are for configuring operation of a patient monitor. The system 10 of the invention may include the patient monitor or may be arranged to be operatively coupled with the patient monitor in use.

In either case the patient monitor unit 14 is preferably a bedside patient monitor unit. It may be a mobile or ambulatory patient monitor unit. For example, it may comprise a trolley supporting components of the patient monitor for easy transport between different locations. The trolley may include castors for wheeling the patient monitor between different locations as needed.

The function of the patient monitor is to display clinical information (related to the monitored patient) in real-time, and in visible and easily interpretable manner (for clinicians).

By way of example, a patient monitor 14 suitable for use in accordance with embodiments of the present invention may include the following main components.

The patient monitor 14 includes connector means or a communication interface for communicatively coupling with a set of patient sensors 56 for receiving sensor data 59. These may include medical biosensors and mechanical sensors. They may also include imaging sensors, e.g. ultrasound or optical sensors. The patient monitor may in some cases include the set of sensors.

The patient monitor 14 includes a signal processing component 16. This component of medical monitors is responsible for converting the signals 59 from the sensors 59 to a format that can be shown on the display device 18 of the patient monitor 14 or transferred to an external display or recording device. It may also perform processing to derive or extract from the incoming data further clinical parameters such as average, maximum or minimum values, or further clinical parameters which are not directly observable.

The patient monitor preferably further includes a local display device 18. This may include one or more digital screens for displaying clinical signals or data. Information displayed on the display device 18 may include acquired 'original' signals as well as computed parameters.

Acquired original signals means signals directly representative of the measurement or parameter monitored by a given sensor. These may be displayed in signal form, e.g. by a waveform indicating values of the parameter over time. In some cases, the displayed signals may not be the raw signals, but may be post-processed by the signal processing component 16, e.g. with one or more filters or amplifiers.

Computed parameters means parameters (from the raw or generated signal data) which are calculated or derived by the signal data, such as statistical values, e.g. maximum, minimum, average, pulse amplitude, frequency or other clinical parameters not directly observable.

In advantageous examples, the patient monitor may further include a communication interface for communicating with one or more remote systems or devices, for instance for communicating the derived information outputs to a central monitoring station arranged for displaying patient data for a plurality of patients, or hospital patient monitoring networks. It may be for communicating with a central server or system for outputting derived information outputs and/or for receiving stored patient medical data, e.g. medical history data about the monitored patient.

The communication interface may make the local patient monitor data available to all connected devices, and may also permit remote control of the patient monitor by remote devices, e.g. enabling control of sensors, display output, storage, and other settings and operations.

In preferred embodiments, the patient monitor may include an alarm means for generating a sensory alarm signal to alert a user, e.g. clinician, based on the derived information outputs meeting one or more alarm criteria, such as exceeding a defined alert threshold. In advantageous examples, the alarm criteria are configured based in part on outputs from the digital twin. For example, one or more thresholds for the alarm trigger may be set based on information from the digital twin.

In some examples, the patient monitor may be arranged to be communicatively coupleable in use with a central display station: This is not directly part of the local patient monitor, since the monitor can operate fully independently without it. However, in many clinical settings, the local patient monitors are connected to a central monitoring station enabling a single staff member to observe and respond to several bedside monitors simultaneously.

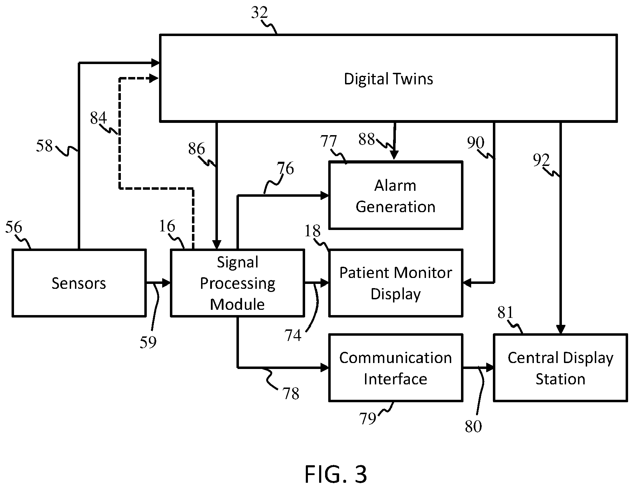

To further illustrate the invention, communication paths between various components of an example system 10 in accordance with one or more embodiments are schematically depicted in Fig. 3. The shown arrangement includes components of both the patient monitor and the processor arrangement and digital twin. However, in further embodiments, the patient monitor may be outside of the system and operatively coupleable to it, as in the example of Fig. 1.

A set of patient sensors 56 are arranged to provide sensor data 58 to the signal processing module 16 of the patient monitor and also provide sensor data 58 to the digital twin 32 (which may be via a further processor arrangement (not shown) or a further communication interface in some examples). In some cases, a different respective set of sensors may feed data to the digital twin and to the signal processing module. More than one digital twin 32 may be provided. For example, if more than one patient is being monitored, at least one digital twin may be provided for each monitored patient.

The signal processing module 16 generates output processed data 74. This may include for example cleaning, filtering, or transformation of the raw sensor data 59, and/or computation of various parameters (e.g. statistical parameters, or additionally medically relevant indicators such as heart rate) from the raw sensor data 59.

The signal processing module 16 can be further connected to an optional alarm generation means 77 for generating a sensory alarm signal to alert a user, e.g. clinician, based on the derived information outputs meeting one or more alarm criteria, such as exceeding a defined alert threshold. The alarm means was discussed above. The signal processing component can provide outputs 76 to the alarm generation means which may be the same as the outputs 74 provided to the display unit 18. The information 76 output may be used by the alarm generator 77 to determine whether an alarm needs to be generated, how it should be generated and/or criteria or parameters for alarm generation. Alternatively, the signal processing module may output trigger signals to the alarm generator 77 for directly triggering generation of an alarm.

A communication interface 79 may be further included. Outputs 78 of the signal processing module 16 can be further provided to the communication interface for communicating to external devices or systems. For example, the clinical information generated by the signal processing module may be communicated as an output 80 to a central display station 81 arranged to display data pertaining to a plurality of patients. Information may also be communicated to a cloud server for example. Features of the communication interface were discussed in more detail above.

Optionally, the output of the signal processing module 16 may also be communicated as a further output 84 to the digital twin 32. This is optional since in principle, all of the signal processing performed by the patient monitor can be performed (with potentially greater accuracy and detail) by the digital twin 32. However, in some cases, for instance where the digital twin is specialized, digital twin simulations may still benefit from the processing output from patient monitor processing module 16. For example, a digital twin specialized for modelling a urinary track may benefit from data input from the patient monitor indicative of current patient SpO₂ level.

As shown, outputs of the digital twin 32 can be provided as inputs to various components for enhancing or augmenting functionality of the patient monitor.

Outputs 86 of the digital twin can be (directly or indirectly, e.g. via a further processor arrangement 22) provided to the signal processing module 16 to guide parameters of the signal processing performed by the signal processing module. This has been discussed in detail above and so will not be described again here for brevity.

Outputs 88 of the digital twin 32 can be used to guide alarm generation by the alarm generation means 77. For example, the digital twin 32 may provide to the alarm generator 77 information 88 indicative e.g. of a current or predicted future state of the patient, predicted pathologies, or more directly of proposed alarm thresholding functions, timing of alarms, and/or intensity of alarms. The alarm generator may configure for instance criteria for the alarm, such as threshold levels for instance, and other settings of the alarm based in part on this information.

Optionally, outputs 90 from the digital twin may be provided directly to the patient monitor for display on the patient monitor display unit 18. The outputs of the digital twin may include for example computed signals and parameters, disease evaluation predictions, current patient status indicators, predictions regarding future patient status, and a variety of other information, as discussed in more detail above. The patient monitor may determine which, if any, of the provided digital twin outputs 90 to display.

In addition, the manner in which the information outputs from the signal processing module and/or from the digital twin 32 are displayed on the display unit 18 may be adapted based on clinical information provided by the digital twin. For example, the signals may be presented as waveforms over defined time windows, e.g. 30-second time windows, or two-minute time windows, based on the feedback received from the digital twin 32. For more clinically relevant parameters, the signal waveform progression over a longer time window can be shown by extending the length of the time axis. For example, if the patient condition is unstable or predicted to be unstable, shorter time domain windows can be used (e.g. in the calculation of the heart rate for instance), allowing faster and more dynamic observations. This feature will be described in greater detail below.

Outputs 92 from the digital twin 32 can also be provided directly to a central display station 81 (where one is present). This can be displayed on the central display station display unit. The outputs may be the same as those 90 provided to the patient monitor, or optionally may be less detailed or expansive.

As mentioned above, according to one or more embodiments, the display of information by the patient monitor display unit may be configured based on outputs from the digital twin 32.

For example, in accordance with one or more embodiments, the selection of clinical information displayed on the patient monitor display 18 may be configured based at least in part on outputs from the digital model 32. In preferred examples, it may be configured based on one or more outputs of the digital twin indicative of a current or future clinical state of the patient.

For example, based on a derived current or future clinical state of the patient, it may be determined that one or more parameters are of more immediate significance than others. The clinical parameters displayed on the screen may be selected to be those determined as the most important. The selection of parameters displayed on the screen may be dynamically updated at regular intervals based on change in patient clinical state as determined by the digital twin.

In accordance with one or more embodiments, the display size at which of each element of clinical information is displayed on the patient monitor display may be determined at least in part on outputs from the digital model. Preferably it is determined based on one or more outputs of the digital model indicative of a current or future clinical state of the patient.

For example, based on a derived current or future clinical state of the patient, it may be determined that one or more parameters are of more immediate significance than others. The more important parameters may be rendered at a larger size on the screen, or otherwise with greater visibility or prominence.

In preferred examples, the display size is proportional to the determined clinical importance of the parameter to the digital twin-determined patient current or future clinical state.

In some examples, the display interface may be configured to provide multiple windows or tabs, each containing different elements of clinical information. In some examples, the window or tab in which each element of clinical information is displayed may be determined at least in part on outputs from the digital model. For example, some windows might be more prominently displayed than others, and/or some tabs might be hidden or minimized by default while others are maximized or visible by default.

Additionally or alternatively, an order in which clinical information is displayed on the screen may be configured at least in part on outputs from the digital model, for example outputs related to a current or future clinical state of the patient. The order may be based on a determined ranking of clinical importance or relevance of the parameters, based on information from the digital twin. For example, the order of display may follow the determined order of importance, with the most important at the highest position on the display and declining in position with determined importance ranking.

In accordance with one or more embodiments, the selection of clinical information displayed on the display of the patient monitor unit and/or the display size at which each element of clinical information is displayed on the patient monitor may be updated recurrently based on changes in said outputs of the digital model.

These features will now be discussed in more detail.

As mentioned above, various parameters and configurations relating to the way in which clinical information is output on the patient monitor display 18 can be either determined and output by the digital twin, or determined based on output from the digital twin 32. By way of example, these display parameters and configurations may include one or more of the following.

Signal ranking and importance indicators for different clinical parameters can be determined based on a current patient status determined by the digital twin. For example, a ranking in terms of clinical significance can be determined and a degree of emphasis or prominence with which the parameter or signal is displayed adapted based on this. For example, the way signals are displayed, and selection of information to be displayed can be determined accordingly.

Signal ranking and importance indicators can be determined for different clinical parameters based on a predicted future status of the patient. For example, the way signals are displayed, and selection of information to be displayed can be adapted, taking into account the predicted future state of the patient. In some examples, signal rankings may additionally or alternatively be determined based on an estimated accuracy level of the output information, e.g. based on a confidence or bias or accuracy indicator of the output information.

Particular features of sensor signals, or particular time windows of patient signals, that are of particular clinical importance can be determined by the digital twin or determined based on its outputs. For example, signal features that are particularly clinically relevant could include amplitude variations, frequency changes, and/or baseline changes. Those particular features or time windows of most importance may be emphasized on the display, e.g. presented with greater prominence, e.g. higher up, at a larger size and/or in a different color or font.

The display layout may be adapted to make space to display additional signal features calculated by the digital twin 32.

Visualization characteristics of displayed clinical information can be adapted based on outputs from the digital twin. For example, adapting the time and amplitude scale of the signal displayed on the patient monitor display 18.

Signal quality indicators may be determined by the digital twin or determined based on its outputs. These can be presented on the patient monitor display, which may trigger the staff to check hardware.

A trajectory of the past, current, and predicted patient status can be determined and displayed based on digital twin 32 outputs. The digital twin 32 is able to predict future patient status, and this can be displayed as a patient condition trajectory on the patient monitor display device 18.

Patient data not available from the patient sensors 56 (e.g. medical image data) may be provided by the digital twin 32 in some examples. For example, the digital twin 32 may have access to additional patient data (electronic health record (EHR) data, medical images, related population data, etc.). If relevant to the current, or predicted near-future condition of the patient (e.g. determined by the processor arrangement 22 or the digital twin 32 itself), such information might also be displayed on the patient monitor display device 18. In some examples, there may be provided interface means for controlling parameters of the digital twin from the patient monitor. For example, a copy of the digital twin user interface may be made available to visualize (and control) from the patient monitor e.g. displayed on the display device 18. This may enable the clinician to control which information is output by the digital twin and to also visualize different digital twin simulation results.

As mentioned above, different clinical parameters or signals output by the signal processing module 16 (or from the digital twin 32) may be ranked in terms of clinical importance, with parameters of their display potentially adjusted based on these rankings.

For example, depending on the patient condition, some signals may be more important (to pay attention to) than the others. In some examples, the digital twin may evaluate the current patient status (or predicted future patient status) and accordingly determine which signals are expected to change. Those signals can be ranked as more important (to pay attention to and monitor) than the others, and therefore they can be displayed differently on the display device. For instance, the order in which the signals are displayed may be adjusted, or more important signals may be displayed in a different color or boldness, or in a different media (e.g. virtual reality if available).

Determination of the signal (importance or significance) rankings can be done in different ways, depending upon how the digital twin 32 is used.

The rankings may be determined and updated in real time, i.e. continuously. This may only be possible if the digital twin 32 is configured to be run in real-time, i.e. the digital twin simulations are run continuously and in real time.

In other cases, the digital twin 32 simulation times may be scheduled to run at regular fixed intervals, for example every 5 minutes. In this case, the signal rankings may be updated whenever an update from digital twin 32 is available, i.e. whenever the digital twin reruns the simulations.

According to one or more examples, the timings of (i.e. regular interval times between) digital twin 32 simulations may be set based on patient condition. Here, instead of using a fixed schedule for digital twin 32 simulation runs, the schedule may be configured to depend upon the patient condition. For example, for stable patients the simulation may be performed with less frequency than for less stable patients. The signal rankings may be updated each time the simulation is updated.

In some cases, the digital twin 32 simulation runs may be configured to be directly triggered by features of the patient condition. For example, in some cases the simulation runs may be triggered based on real-time sensor signals, for example based on exceeding one or more thresholds. For example, if heart rate exceeds a defined percentage of the average heart rate, or a fixed value, then a new digital twin 32 simulation is run (and signal ranking is updated).

The digital twin 32 simulation timings may be configured based on features of patient care. For example, in this case, the digital twin 32 simulations may be triggered based on the activities of the patient, or the care that the patient is receiving. Such activities might include for instance: drug intake, food intake, toilet visit, position change in bed, motion in bed, urination, talking, coughing, other sounds (e.g. indicating pain or breathing difficulty), and/or location change. These patient activities might be detected automatically using sensors, for example optical sensors (e.g. camera), motion sensors (e.g. accelerometers), thermal sensors, sound sensors, and/or presence detection sensors (e.g. indoor GPS, PIR, Wi-Fi).

Digital twin 32 simulations may be initiated by a caregiver. The digital twin 32 simulations may be triggered by caregivers, in a manual manner. For example, after or before administration of a drug, the caregiver might trigger a digital twin 32 simulation to evaluate clinical parameter characteristics (i.e. to visualize which signals are expected to change, and are therefore critical to pay attention to).

In some examples, the occurrence of manual triggering of a simulation run by a user (e.g. caregiver) may be provided as one of the outputs of the digital twin. The signal processing and/or display settings may be configured at least in part based on this output.

Digital twin 32 simulations may be dependent on caregiver activity. The activity (e.g. motion, presence, communication, schedule) of caregivers may be monitored in an automated manner (based on one or more sensors), and can be used to initiate or plan the simulations. The monitoring of caregivers can be achieved in some cases based on environment sensors (positioned in the room) or for instance wearable sensors (attached to the caregiver).

Digital twin simulation timings may be triggered based on environment sensors. Sensors may be provided monitoring environmental conditions such as air quality, temperature, noise level, number of people present in the room, and a wide range of other parameters. One or more of these may in some cases be used to trigger digital twin 32 simulations.

According to one or more embodiments, the timings of digital twin 32 simulations may be dynamically configured by the digital twin itself, e.g. based on outputs of the digital twin. For example, the digital twin may determine one or more trigger criteria related to patient condition for triggering simulation runs, e.g. a particular threshold change in one or more clinical parameters for instance. For example, for a stable patient, only drug intake related simulations may be the most relevant, while for a post-operative patient (at risk of hemorrhage), any changes related to ECG, blood pressure, or motion may be relevant. In other words, based on a first digital twin 32 simulation, the relevant trigger criteria for triggering a new simulation run (such as change points of one or more parameters) are determined, and then a next digital twin 32 simulation is activated when the determined criteria are satisfied. Thus timings are configured dynamically using outputs of the digital twin itself.

As mentioned above, and with reference to Fig. 3, outputs can be provided from the digital twin 32 to the patient monitor unit (outputs 86, 90) and to a central display station (output 92) (where one is present). Each of these units may be operable to select, in some examples, which of the information output by the digital twin to display.

In some examples, the central display unit 81 may place greater emphasis on outputs of the digital twin 32 indicative of a predicted (future) state of the patient. In other words, while bedside monitor display 18 may more often display real-time signals, the central monitor may give more priority to the predicted future information.

In some examples, in case the patient condition is predicted to worsen, a message indicative of this can be displayed on the central display station 81 (and optionally an alarm may be generated). Based on this, the staff can be alerted in advance, enabling the staff to be physically (or virtually) present when the condition does worsen. Staff monitoring the patient during and following the alert can enable timely and more accurate interventions, as well as new observations (by the staff) related to the patient condition.

In some examples, the signals and information displayed on the monitors may be adapted based on an expertise level of a person who is attending to the patient, or present with them in the room. This may be a staff member who is examining, or expected to examine, the patient, or it may be a relative or outside caregiver.

For example, if a ward nurse is examining the patient, a simplified view of a clinical signal, together with the digital twin 32 derived current and predicted future condition of the patient, might be displayed. In case a surgeon is examining the patient, the display screen may include all clinical signals, as well as various treatment options determined by the digital twin 32.

Detection of qualifications levels of visiting persons may be performed automatically, for example using indoor positioning sensors, and by making reference to a dataset of different staff expertise. When the patient is alone, or with relatives, then the information displayed might be very general or basic, for example only indicating if the patient condition is stable (not requiring immediate attention), or is (or is predicted to soon be) unstable.

Adapting the displayed information based on the staff or visitor characteristics also enables protection of the patient privacy.

In accordance with one or more embodiments, the set of patient sensors 56 may include one or more wearable or portable sensors mounted to the patient. These may include for instance an ECG monitor for cardiac monitoring. There might also be for example a wearable or mobile treatment device, such as one or more medication patches for continuously providing medications for Parkinson's disease patients (e.g. patches providing continuous transdermal delivery of a dopamine agonist). In these cases, the digital twin 32 may be arranged to directly control the settings of these sensors and/or devices, based on the current and predicted patient status determined by the digital twin. For example, it has been shown that adapting the sensitivity improves atrial fibrillation detection using Holter monitor data. The digital twin 32 may be used to determine when and how the sensitivity can be adapted.

In addition, for such (mobile monitoring) sensors, or mobile devices, battery, storage and memory capacity considerations may become relevant. The digital twin 32 may be used to determine the most clinically relevant signals (and signal features) to acquire, process, and store at any given moment based on detected patient condition, resulting in optimization of the power, processing and memory resources.

As discussed above, embodiments of the invention make use of a processor arrangement and/or processing component to perform data processing. The processor arrangement may comprise one or more processors. Also, the signal processing component 16 of the patient monitor unit 14 may comprise one or more processors. Such processors can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples in accordance with a further aspect of the invention also provide a method of configuring patient monitoring by a patient monitor unit, the patient monitor unit arranged in use to receive input sensor data from one or more patient sensors, and operable to display clinical information related to a monitored patient based on the sensor data, the patient monitor unit comprising a signal processing module configured to apply signal processing to the input sensor data to generate one or more information outputs related to the monitored patient, and the method comprising retrieving a digital model of at least part of an anatomy of the monitored patient, and developing the model based on input sensor data from one or more patient sensors so as to simulate within the model an actual physical state of said at least part of the anatomy of the patient.

In advantageous embodiments, the method comprises the signal processing performed by the signal processing module based at least in part on outputs from the digital model.

Examples of circuitry that may be employed in various embodiments of the present disclosure include, but are not limited to, conventional microprocessors, application specific integrated circuits (ASICs), and field-programmable gate arrays (FPGAs).

In various implementations, the processor arrangement may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A system (10) for configuring patient monitoring by a patient monitor unit (14),
the patient monitor unit arranged in use to receive input sensor data from one or more patient sensors (56), and operable to display clinical information related to a monitored patient based on the sensor data, the patient monitor unit (14) comprising a signal processing module (16) configured to apply signal processing to the input sensor data to generate one or more information outputs related to the monitored patient,
the system (10) comprising:
a processor arrangement (22) communicatively coupled to a data storage arrangement (30) storing a digital model (32) of at least part of an anatomy of the monitored patient, and further coupleable in use with one or more patient sensors (56) to receive patient sensor data, and the digital model configured for simulating an actual physical state of said at least part of the anatomy based on development of the digital model (32) using the sensor data;
wherein the processor arrangement (22) is adapted to configure the signal processing performed by the signal processing module (16) based at least in part on outputs from the digital model (32).

2. A system as claimed in claim 1, wherein the selection of information outputs to be derived by the signal processing module (16) is determined based on outputs from the digital model (32).

3. A system as claimed in claim 1 or 2, wherein a selection of one or more signal processing methods applied for deriving a given one or more information outputs is determined based in part on outputs from the digital model (32).

4. A system as claimed in any of claims 1-3, wherein the digital model (32) is operable to generate one or more outputs related to a current or predicted future clinical state of the at least part of the anatomy of the patient, and optionally wherein the digital model is operable to generate one or more outputs related to a current or predicted future pathology of the at least part of the anatomy of the patient, and in either case wherein the signal processing is configured based at least in part on said outputs.

5. A system as claimed in any of claims 1-4, wherein:
the digital model (32) is operable to generate one or more prediction outputs pertaining to a predicted future state of the at least part of the anatomy, and wherein the signal processing is configured based at least in part on said prediction outputs; and/or
the digital model is operable to generate one or more prediction outputs indicative of any predicted future pathology of said at least part of the anatomy, and wherein the signal processing is configured such that the information outputs relate to said predicted future pathology.

6. A system as claimed in any of claims 1-5, wherein the digital model (32) is operable to generate one or more outputs indicative of physiological or anatomical parameters of the patient, and wherein the clinical information displayed by the patient monitor is based in part on said outputs of the digital model.

7. A system as claimed in any of claims 1-6, wherein the information outputs generated by the signal processing module (16) include one or more clinical parameters of the patient, for example one or more physiological or anatomical parameters of the patient.

8. A system as claimed in any of claims 1-7, wherein one or more of the information outputs generated by the signal processing module (16) are supplied as respective information inputs to the digital model (32).

9. A system as claimed in any of claims 1-8, wherein a selection of clinical information displayed on the patient monitor display (18) is configured based at least in part on outputs from the digital model (32), and preferably configured based on one or more outputs of the digital model indicative of a current or future clinical state of the patient.

10. A system as claimed in any of claims 1-9, wherein a display size at which each element of clinical information is displayed on the patient monitor display (18) is determined at least in part on outputs from the digital model, and preferably determined based on one or more outputs of the digital model indicative of a current or future clinical state of the patient.

11. A system as claimed in claim 9 or 10, wherein the selection of clinical information displayed by the patient monitor unit (14) and/or the display size at which each element of clinical information is displayed by the patient monitor unit (14) is updated recurrently based on changes in said outputs of the digital model (32).

12. A system as claimed in any of claims 1-11, wherein a frequency of simulation runs of the digital model (32) is adjusted recurrently based in part on outputs of the digital model, for example based on one or more outputs indicative of a current or predicted future clinical state of the patient determined by the digital model.

13. A system as claimed in any of claims 1-12, wherein the system comprises the patient monitor unit (14).

14. A method of configuring patient monitoring by a patient monitor unit (14),
the patient monitor unit (14) arranged in use to receive input sensor data (59) from one or more patient sensors (56), and operable to display clinical information related to a monitored patient based on the sensor data, the patient monitor unit comprising a signal processing module (16) configured to apply signal processing to the input sensor data to generate one or more information outputs related to the monitored patient,
and the method comprising:
retrieving a digital model (32) of at least part of an anatomy of the monitored patient, and developing the digital model based on input sensor data from one or more patient sensors so as to simulate with the model an actual physical state of said at least part of the anatomy of the patient; and
configuring the signal processing performed by the signal processing module (16) based at least in part on outputs from the digital model (32).

15. A computer program product comprising code means configured when run on a processor, to cause the processor to perform the method of claim 14.
